# EUROPÄISCHE PATENTSCHRIFT

(11) **EP 1 877 358 B1**
(45) Veröffentlichungstag und Bekanntmachung des Hinweises auf die Patenterteilung: **17.12.2008**
(21) Anmeldenummer: 06754896.6
(22) Anmeldetag: 27.04.2006
(51) Int. Cl.: C07C 51/31, C07C 55/24

(54) **VERFAHREN ZUR HERSTELLUNG VON TETRACARBONSÄUREN**
METHOD FOR PRODUCING TETRACARBOXYLIC ACIDS
PROCEDE DE PRODUCTION D'ACIDES TETRACARBOXYLIQUES

(30) Priorität: 29.04.2005 DE 102005020494
(43) Veröffentlichungstag der Anmeldung: 16.01.2008
(73) Patentinhaber: BASF SE, 67056 Ludwigshafen (DE)
(72) Erfinder: GUMLICH, Kai, 68159 Mannheim (DE); TELES, Joaquim Henrique, 67166 Otterstadt (DE)
(86) Internationale Anmeldenummer: PCT/EP2006/061887
(87) Internationale Veröffentlichungsnummer: WO 2006/117326

(56) Entgegenhaltungen:
- EP-A- 0 039 048
- EP-A- 0 688 897
- US-A- 5 157 152

## Beschreibung

Die vorliegende Erfindung betrifft ein Verfahren zur Herstellung von Tetracarbonsäuren der allgemeinen Formel I 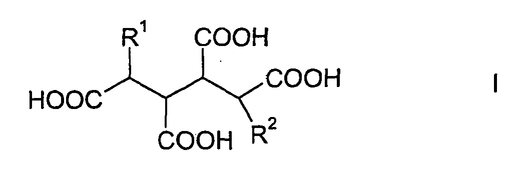 oder ihrer Salze, dadurch gekennzeichnet, dass man mindestens eine Verbindung der allgemeinen Formel II a oder II b 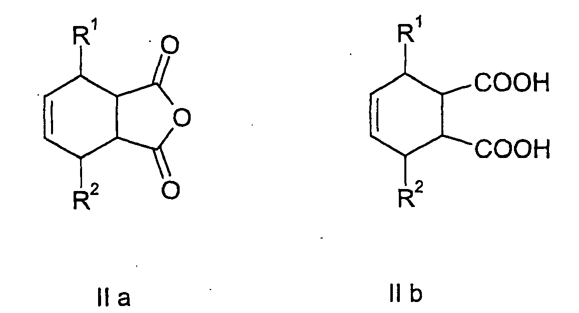
(a) zunächst mit mindestens einem Epoxidierungsreagenz umsetzt und
(b) danach mit Salpetersäure oder mindestens einem Stickoxid oxidiert,
wobei die Variablen wie folgt definiert sind:
- R¹, R²: gleich oder verschieden und gewählt aus Wasserstoff, Phenyl und C₁-C₁₀-Alkyl.

Tetracarbonsäuren finden neuerdings zahlreiche Anwendungen nicht nur allgemein als schwerflüchtige organische Säuren, sondern auch als Mittel zur formaldehydfreien Ausrüstung und insbesondere zur formaldehydfreien Knitterfestausrüstung von Textilien , s. beispielsweise EP-A 0 354 648.

Bisher bekannte Verfahren zur Herstellung von Tetracarbonsäuren beruhen üblicherweise auf der Oxidation von Diels-Alder-Addukten von Dienen wie beispielsweise Butadien und von Dicarbonsäureanhydriden wie beispielsweise Maleinsäureanhydrid. Bekannte Verfahren weisen jedoch im Allgemeinen Nachteile auf. So beschreibt US 5,157,152 ein zweistufiges Verfahren zur Oxidation von beispielsweise Tetrahydrophthalsäure zu Butan-1,2,3,4-tetracarbonsäure durch zweimalige Umsetzung mit Wasserstoffperoxid bei jeweils 80 bis 115°C, wobei als Zwischenstufe 4,5-Dihydroxyhexahydrophthalsäure entsteht. Man beobachtet jedoch, dass Verunreinigungen wie die als Katalysator eingesetzten Wolframverbindungen nur schlecht abgetrennt werden können und dass die Umsetzung nicht vollständig ist: es verbleibt 4,5-Dihydroxyhexahydrophthalsäure, so genanntes Glykol, zu einem signifikanten Anteil im Produkt. Auch ist eine große Menge Wasserstoffperoxid einzusetzen. So sind mindestens 4,1 Äquivalente Wasserstoffperoxid erforderlich, um 4,5-Dihydroxyhexahydrophthalsäure zu Butan-1,2,3,4-tetracarbonsäure zu oxidieren.

Aus J.E. Franz et al., J. Org. Chem. 1965, 30, 1488 ist bekannt, dass man *cis*-Δ⁴-Tetrahydrophthalsäure in einem Einstufenverfahren zu Butan-1,2,3,4-tetracarbonsäure oxidieren kann, indem man *cis*-Δ⁴-Tetrahydrophthalsäure zu einer Lösung von Ammoniummetavanadat in konzentrierter Salpetersäure gibt (Beispiele rechte Spalte, Seite 1491). Die Ausbeute an Butan-1,2,3,4-tetracarbonsäure lässt sich jedoch verbessern.

Aus EP-A-0 039 048 ist ein Verfahren zur Oxidierung von Tetrahydrophthalsäure zu Butan-1,2,3,4-tetracarbonsäure mit Ammoniummetavanadat in konzentrierter Salpetersäure bekannt.

Aus EP-A-0 688 897 ist ein Verfahren zur Oxidierung von Tetrahydrophthalsäure oder Tetrahydrophthalsäureanhydrid zu Butan-1,2,3,4-tetracarbonsäure durch Oxidierung mit Wasserstoffperoxid bekannt.

Aus DE 30 16 225 ist bekannt, dass man beispielsweise Butan-1,2,3,4-tetracarbonsäure, die man durch Oxidation von Δ⁴-Tetrahydrophthalsäure mit Ammoniummetavanadat in konzentrierter Salpetersäure in einem Einstufenverfahren gewonnen hat, üblicherweise verunreinigt ist mit Nitroverbindungen bzw. Stickstoffverbindungen (Seite 3, Zeile 19 ff., Seite 4, Zeile 5 ff.), die bei späterer Verwendung stören können. Beispielsweise führen derartige Verunreinigungen bei der Veresterung zu unerwünschter Braunfärbung. DE 30 16 225 zeigt, dass man beispielsweise Butan-1,2,3,4-tetracarbonsäure durch mehrstündige Nachbehandlung mit Salpetersäure aufreinigen kann. Ein derartiges Verfahren ist jedoch zeitaufwändig, und die Ausbeute ist mit 66,5% verbesserungsfähig.

Es bestand also die Aufgabe, ein Verfahren bereit zu stellen, das Tetracarbonsäuren in guter Reinheit und guter Ausbeute bei vertretbarem Aufwand liefert. Es bestand weiter die Aufgabe, Tetracarbonsäuren in guter Reinheit bereit zu stellen, so dass sie sich zur Verwendung für die Knitterfestausrüstung von Textilien eignen.

Demgemäß wurde das eingangs definierte Verfahren gefunden.

Das erfindungsgemäße Verfahren geht aus von einer oder mehreren Verbindungen der allgemeinen Formel II a oder II b 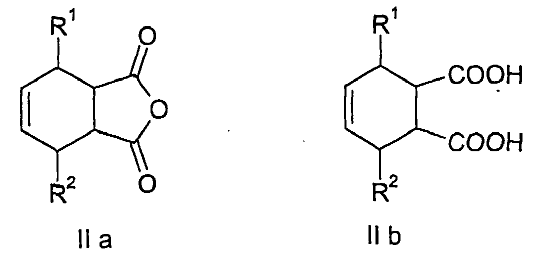 in der die Variablen wie folgt definiert sind:
- R¹, R²: sind verschieden oder vorzugsweise gleich und gewählt aus Phenyl,
C₁-C₁₀-Alkyl, verzweigt oder vorzugsweise unverzweigt, wie beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, 2-Ethylhexyl, n-Nonyl, n-Decyl, besonders bevorzugt unverzweigtes C₁-C₄-Alkyl wie Methyl, Ethyl, n-Propyl, n-Butyl,
und besonders bevorzugt Wasserstoff.

Ganz besonders bevorzugt sind R¹ und R² gleich und jeweils Wasserstoff.

Zur Durchführung des erfindungsgemäßen Verfahrens kann man auch von Gemischen von mindestens einer Verbindung der Formel II a und mindestens einer Verbindung der Formel II b ausgehen. Beispielsweise ist es möglich, von Gemischen von 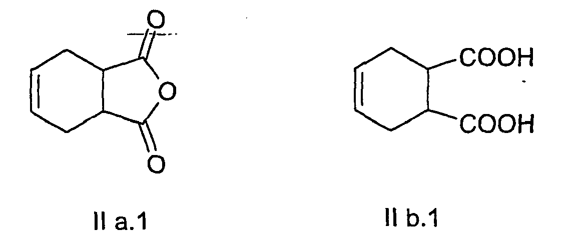 auszugehen. In Verbindungen der allgemeinen Formel II b ist die relative Stereochemie der beiden Carboxylgruppen zueinander offen. Vorzugsweise stehen die beiden Carboxylgruppen in *cis*-Konfiguration zueinander.

Verbindungen der allgemeinen Formel II a und II b sind als solche bekannt und nach an sich bekannten Methoden erhältlich, beispielsweise durch Diels-Alder-Reaktion von Dienen und Dicarbonsäuren bzw. deren Anhydriden.

In Schritt (a) setzt man mindestens eine Verbindung der allgemeinen Formel II a bzw. II b zunächst mit mindestens einem Epoxidierungsreagenz um.

Als Epoxidierungsreagenzien sind insbesondere Peroxide geeignet, beispielsweise organische Peroxide wie tert.-Butylhydroperoxid und meta-Chlorperbenzoesäure, und ganz besonders bevorzugt Wasserstoffperoxid. Wünscht man Wasserstoffperoxid einzusetzen, so setzt man es beispielsweise als wässrige Lösung ein, bevorzugt als 10-bis 70 Gew.-% wässrige Lösung, besonders bevorzugt als 30- bis 50 Gew.-% wässrige Lösung.

Man kann Epoxidierungsreagenz (a) äquimolar, bezogen auf Verbindung der allgemeinen Formel II a bzw. II b, einsetzen.

Epoxidierungsreagenz setzt man in Schritt (a) vorzugsweise in einem Überschuss ein. Darunter ist ein Überschuss bezogen auf Verbindung der allgemeinen Formel II a bzw. II b zu verstehen. Geeignete Überschüsse sind insbesondere 5 mol-% bis 300 mol-%, bevorzugt 100 bis 150 mol-%.

Die Umsetzung von Verbindung der allgemeinen Formel 11 a bzw. II b mit Epoxidierungsreagenz in Schritt (a) kann beispielsweise bei einer Temperatur im Bereich von 30 bis 150°C, bevorzugt von 60 bis 105°C, besonders bevorzugt 70 bis 90°C erfolgen.

Die Umsetzung von Verbindung der allgemeinen Formel II a bzw. II b mit Epoxidierungsreagenz in Schritt (a) kann vorzugsweise bei Normaldruck erfolgen. Es ist jedoch auch möglich, bei erhöhtem Druck wie beispielsweise 1,1 bis 5 bar zu arbeiten.

Die Umsetzung von Verbindung der allgemeinen Formel II a bzw. II b mit Epoxidierungsreagenz in Schritt (a) kann beispielsweise über einen Zeitraum von 30 Minuten bis 10 Stunden durchführen, bevorzugt sind eine bis fünf Stunden.

Die Reihenfolge der Zugabe von Verbindung der allgemeinen Formel II a bzw. II b und Epoxidierungsreagenz ist an sich unkritisch. In einer Ausführungsform der vorliegenden Erfindung geht man so vor, dass man Verbindung der allgemeinen Formel II a bzw. II b, gegebenenfalls mit einem inerten Verdünnungsmittel vermischt, vorlegt und mit Epoxidierungsreagenz versetzt.

Zur Umsetzung von Verbindung der allgemeinen Formel II a bzw. II b mit Epoxidierungsreagenz in Schritt (a) geht man in einer Ausführungsform der vorliegenden Erfindung so vor, dass man Verbindung der allgemeinen Formel II a bzw. II b zunächst mit einem inerten Verdünnungsmittel vermischt, insbesondere mit Wasser, beispielsweise mit 10 bis 1000 Gew.-% Wasser, bezogen auf Verbindung der allgemeinen Formel II a bzw. II b. Zum Vermischen kann man beispielsweise bei Temperaturen im Bereich von 20 bis 150°C, bevorzugt 80 bis 100°C arbeiten.

In einer Ausführungsform der vorliegenden Erfindung arbeitet man in Schritt (a) unter Verwendung von mindestens einem Katalysator. Geeignete Katalysatoren sind beispielsweise Brönsted-Säuren, Wolframate, Molybdate, Heterpolyverbindungen des Molybdäns oder Wolframs und insbesondere Molybdän- oder Wolframverbindungen wie beispielsweise Natriummolybdat oder Ammoniumwolframat. Vorzugsweise arbeitet man in Schritt (a) ohne Katalysator.

Die Reaktionswärme aus Schritt (a) kann man beispielsweise durch indirekte Kühlung abführen oder dadurch, dass man während der Umsetzung inertes Verdünnungsmittel und/oder gebildetes Wasser partiell oder vollständig abdestilliert.

Durch die Umsetzung in Schritt (a) erhält man zunächst Epoxid der allgemeinen Formel III a oder III b 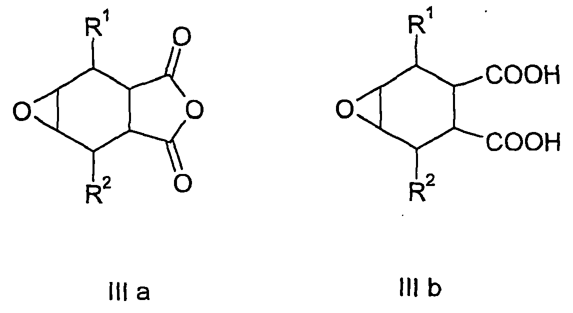 beispielsweise als Gemisch, insbesondere was die Konfiguration des Epoxids zur Anhydridgruppierung bzw. den Carboxylgruppen und den Resten R¹ und R² für den Fall betrifft, dass R¹ und R² ungleich Wasserstoff sind. Arbeitet man in Gegenwart von Wasser, so werden üblicherweise Anhydridgruppierung und Epoxid *in situ* beispielsweise partiell oder auch quantitativ hydrolysiert, und man erhält Verbindung der allgemeinen Formel IV 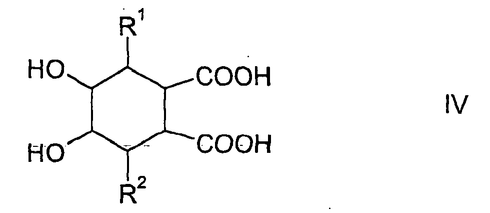 in der die Hydroxylgruppen üblicherweise trans zueinander stehen und die im Folgenden auch als Zwischenprodukt bezeichnet wird.

Man kann Verbindung der allgemeinen Formel IV isolieren. Es ist jedoch bevorzugt, auf eine Isolierung und somit Aufreinigung von Verbindung der allgemeinen Formel IV zu verzichten. In einer Ausführungsform der vorliegenden Erfindung schließt man die Schritte (a) und (b) ohne Isolierung von Zwischenprodukts an.

In Schritt (b) oxidiert man mit Salpetersäure, beispielsweise 20 bis 100 Gew.-% wässriger Salpetersäure, vorzugsweise mit 50 bis 70 Gew.-%, besonders bevorzugt 65 bis 68 Gew.-% wässriger Salpetersäure, oder mit mindestens einem Stickoxid wie beispielsweise N₂O₅.

Schritt (b) kann man ohne Katalysator durchführen.

In einer Variante der vorliegenden Erfindung oxidiert man in Schritt (b) in Gegenwart von mindestens einem Katalysator.

Bei Katalysator im Sinne der vorliegenden Erfindung kann es sich um eine oder mehrere gelöste Verbindungen handeln, die in Salpetersäure löslich sind und die man separat oder zusammen mit Salpetersäure dosieren kann. Man kann in einer Variante der vorliegenden Erfindung Katalysator für Schritt (b) auch bereits bei Beginn von Schritt (a) dosieren.

Katalysatoren im Sinne der vorliegenden Erfindung sind vorzugsweise Wolfram-frei, d.h. es handelt sich um Verbindungen von beispielsweise Mangan und besonders bevorzugt Vanadium. Der Gehalt an Wolfram überschreitet, wenn Wolfram als Verunreinigung in erfindungsgemäß in Schritt (b) eingesetztem Katalysator vorliegt, 10 Gew.-% an Katalysator nicht. Ganz besonders bevorzugt handelt es sich bei Katalysator im Sinne der vorliegenden Erfindung um eine oder mehrere Verbindungen, die Vanadium enthält bzw. enthalten.

In einer Ausführungsform der vorliegenden Erfindung verwendet man vorzugsweise in Schritt (b) einen Mischkatalysator, der eine katalytisch aktive Hauptkomponente enthält wie beispielsweise eine Vanadiumverbindung und einen Cokatalysator, der als einziger Katalysator genommen nur geringe katalytische Aktivität aufweist. Als Cokatalysatoren sind insbesondere Eisen- und Kupfersalze geeignet, beispielsweise FeCl₃, CuSO₄ und CuCl₂.

In einer Ausführungsform der vorliegenden Erfindung verwendet man eine Verbindung, die mindestens ein Atom Vanadium pro Molekül enthält, zusammen mit 1 bis 1,5 Moläquivalent Cokatalysator, bezogen auf Vanadiumverbindung, beispielsweise FeCl₃, oder Cu-Salze wie beispielsweise CuSO₄ oder CuCl₂.

Dabei kann man beispielsweise Vanadiumverbindungen einsetzen, bei denen Vanadium in beliebiger Oxidationsstufe vorliegt, vorzugsweise aber in der Oxidationsstufe +5.

Besonders bevorzugte Katalysatoren sind Heteropolysäuren des Vanadiums und Ammoniummetavanadat.

In einer Ausführungsform der vorliegenden Erfindung setzt man im Bereich von 0,001 bis 1 Gew.-%, bevorzugt 0,01 bis 0,1 Gew.-% und besonders bevorzugt 0,03 bis 0,05 Gew.-% Katalysator ein, bezogen auf Verbindung der allgemeinen Formel 11 a bzw. II b.

Man kann zum Starten der Oxidation in Schritt (b) mindestens einen Starter zugeben. Geeignete Starter sind beispielsweise Kaliumnitrit und besonders bevorzugt Natriumnitrit. Beispielsweise kann man 0,01 bis 0,1 Gew.-% Starter zugeben, bezogen auf Verbindung der allgemeinen Formel II a bzw. II b, bevorzugt sind 0,03 bis 0,05.

Die Oxidation in Schritt (b) kann man beispielsweise bei Temperaturen in einem Bereich von 20 bis 150°C, bevorzugt bei 50 bis 90°C, besonders bevorzugt bis 70°C durchführen.

Die Oxidation in Schritt (b) kann man beispielsweise bei Normaldruck durchführen. Es ist auch möglich, die Oxidation in Schritt (b) bei einem Druck im Bereich von 1,1 bis 10 bar durchzuführen.

In einer Ausführungsform der vorliegenden Erfindung verwendet man Salpetersäure oder Stickoxid wie beispielsweise N₂O₄ oder N₂O₅ in einem Überschuss, bezogen auf Zwischenprodukt. Geeignet sind beispielsweise 2 bis 10 Äquivalente, bevorzugt 3 bis 7 Äquivalente, besonders bevorzugt 3 bis 6 Äquivalente Salpetersäure oder Stickoxid wie beispielsweise N₂O₅, bezogen auf Zwischenprodukt.

In einer Ausführungsform der vorliegenden Erfindung führt man Schritt (a) und Schritt (b) unter Inertgas durch. Geeignete inertgase sind beispielsweise Edelgase und Stickstoff. Es ist jedoch auch möglich, unter Luft zu arbeiten.

In vielen Fällen entstehen bei der Oxidation in Schritt (b) nitrose Gase (NOₓ), die mit Inertgas oder Luft ein Gasgemisch bilden. Es ist bevorzugt, im Verlauf der Durchführung von Schritt (b) des erfindungsgemäßen Verfahrens entstandene nitrose Gase aus dem Abgas zu entfernen.

Grundsätzlich kommen zur Entfernung der nitrosen Gase alle bekannten Verfahren zur Abtrennung von NOₓ in Frage. Geeignet sind beispielsweise die katalytische Reduktion mit Kohlenwasserstoffen oder Ammoniak, die katalytische Zersetzung an geeigneten Katalysatoren, Absorption in Abgas-oxidierenden Lösungen sowie die Absorption in sauren oder alkalischen Lösungen.

Geeignete Abgas-oxidierende Lösungen im Rahmen der vorliegenden Erfindung sind beispielsweise Lösungen von Wasserstoffperoxid. Als stark saure Lösungen sind beispielsweise Lösungen enthaltend Salpetersäure oder Schwefelsäure geeignet. Als alkalische Lösungen eignen sich beispielsweise wässrige Lösungen von Hydroxiden oder Carbonaten, beispielsweise Natriumhydroxid oder Natriumcarbonat. Als Flüssigkeiten für diese Wäsche eignen sich neben den bereits genannten insbesondere diejenige, die zur Entfernung von NOₓ aus Abgasen an sich bekannt sind, beispielsweise wässrige Lösungen oder Suspensionen enthaltend Magnesiumcarbonat, Magnesiumhydroxid, Lösungen von Vanadiumverbindungen in salpetriger Säure, Ammoniumsulfid und Ammoniumbisulfid, Kalkwasser, Ammoniak, Wasserstoffperoxid und insbesondere Lösungen enthaltend Natriumcarbonat, Natriumbicarbonat oder Natriumhydroxid.

Geeignete Verfahren sind beispielsweise genannt in M. Thiemann et al. in Ullmann's Encyclopedia, 6th Edition, 2000, Electronic Edition, Kapitel "Nitric Acid, Nitrous Acid, and Nitrogen Oxides", Abschnitt 1.4.2.3.

Allgemein erfolgt die NOₓ-Absorption in Einrichtungen, in denen eine Gas-Flüssig-Phasengrenzfläche vorliegt, über die ein Stoff- und Wärmeübergang zwischen den Phasen ermöglicht wird, und die bei Bedarf mit internen oder externen Einrichtungen zur Wärmezufuhr und/oder Wärmeabfuhr versehen sind.
Die Führung der Phasen im Absorber kann im Gleichstrom, im Gegenstrom oder einer Kombination der genannten erfolgen.

Die Absorption kann erfindungsgemäß ein- oder mehrstufig durchgeführt werden.

Die Absorption erfolgt erfindungsgemäß bei Temperaturen zwischen -20 und 100 °C, bevorzugt zwischen 0 und 60 °C, besonders bevorzugt zwischen 0 und 40 °C und bei Drücken zwischen 0,1 und 100 bar, bevorzugt zwischen 1 und 30 bar.

Mögliche Ausführungsformen des Absorbers sind Kolonnen mit Böden, beispielsweise Glockenböden oder Siebböden, Kolonnen mit strukturierten Einbauten wie beispielsweise Packungen, Kolonnen mit unstrukturierten Einbauten wie beispielsweise Füllkörpern, oder Apparate in denen die Flüssigphase dispergiert vorliegt, beispielsweise durch Versprühen in Düsen, oder eine Kombination der vorstehend genannten Absorbern.

Die Abtrennung von NOₓ erfolgt bevorzugt durch Absorption in einer sauren oder einer alkalischen Lösung. Die Absorption wird zwischen -20 und 120°C, insbesondere zwischen -10 und 75°C, bevorzugt zwischen 0 und 60°C, beispielsweise zwischen 0 und 40 °C und bei einem Druck zwischen 0,2 und 100 bar, insbesondere zwischen 0,5 bis 50 bar, bevorzugt zwischen 1 und 10 bar durchgeführt.

Sofern die NOₓ-Konzentration im Abgas mehr als 1 Vol.-% beträgt, wird als Abgas-oxidierende Lösung bevorzugt wässrige Salpetersäure eingesetzt, mit einem HNO₃-Gehalt zwischen 0,1 und 69 Gew.-%, bevorzugt zwischen 1 und 10 Gew.-%. Vorteilhaft ist dabei, dass die NOₓ-Abreicherung in der Gasphase mit der Herstellung von Salpetersäure mit 1 bis 69 Gew.-% HNO₃ einhergeht. Im Sinne der weiteren Nutzbarkeit wird dabei bevorzugt eine Salpetersäure mit 30 bis 60 Gew.-% HNO₃ hergestellt.

Gemäß einer Ausführungsform der vorliegenden Erfindung kann an die Abgaswäsche anschließen, vorzugsweise eine chemische Wäsche, besonders bevorzugt mit Natriumcarbonat-Lösung oder Natronlauge.

Im Rahmen der vorliegenden Erfindung kann z.B. die chemische Carbonat-Wäsche durch eine selektive katalytische Reduktion mit Ammoniak ersetzt werden, bei der sich N₂O inert verhält. Diese sogenannte SCR-DeNOx- oder DeNOx-Technologie wird beispielsweise in Ullmann's Encyclopedia of Chemical Technology, Kapitel "Air", Abschnitt 7.2.3.1. "Catalytic Reduction of Nitrogen Oxides in Flue Gases and Process Off-Gases" von J. Wolf et al., 6. Ausgabe (Online Edition), 2000 beschrieben. Gemäß dieser bevorzugten Ausführungsform der vorliegenden Erfindung können NOₓ-Konzentrationen von weniger als 100 ppm, vorzugsweise weniger als 50 ppm, beispielsweise weniger als 25 ppm und besonders bevorzugt von bis zu 5 ppm erreicht werden.

Es ist in einer speziellen Variante der vorliegenden Erfindung möglich, entstehende nitrose Gase mit Hilfe eines basischen Gaswäschers, beispielsweise mit Hilfe eines mit Natronlauge gefüllten Abgaswäschers, aus dem Gasgemisch zu entfernen.

Die Reihenfolge der Zugabe von Salpetersäure, Zwischenprodukt und Katalysator und gegebenenfalls Starter ist an sich unkritisch. Bevorzugt ist es jedoch, Salpetersäure, Katalysator und gegebenenfalls Starter zu Zwischenprodukt, gegebenenfalls vermischt mit Wasser aus Schritt (a), zu geben.

In einer Ausführungsform der vorliegenden Erfindung vermischt man Salpetersäure, Zwischenprodukt, gegebenenfalls vermischt mit Wasser aus Schritt (a), und Katalysator und gegebenenfalls Starter bei Temperaturen im Bereich von 20 bis 100°C; bevorzugt 50 bis 90°C, besonders bevorzugt 65 bis 75°C.

In einer Ausführungsform der vorliegenden Erfindung führt man Schritt (b) über einen Zeitraum im Bereich von 1 Stunde bis 10 Stunden, bevorzugt bis 5 Stunden durch.

Im Anschluss an Schritt (a) und (b) kann man aufarbeiten. Dazu trennt man gewünschte Verbindung der allgemeinen Formel I als solche oder in Form eines ihrer Salze, bevorzugt als Monoalkalimetallsalz, aus der entstandenen Reaktionsmischung ab.

Als Salze von Verbindung der allgemeinen Formel I sind beispielsweise Salze von Alkalimetallen wie Lithium, Rubidium und insbesondere Kalium und Natrium, insbesondere Monoalkalimetallsalze wie beispielsweise Mononatriumsalze und Monokaliumsalze zu nennen.

In einer Ausführungsform der vorliegenden Erfindung kann man aufarbeiten, indem man zunächst abkühlen lässt, beispielsweise auf Temperaturen im Bereich von 25 bis 40°C oder auf Zimmertemperatur, und danach durch geeignete Fest-flüssig-Trennungsmethoden sich abscheidende Verbindung der allgemeinen Formel I abtrennt, insbesondere durch Dekantieren oder durch Filtration. Nach der Abtrennung von Verbindung der allgemeinen Formel I kann man erfindungsgemäß hergestellte Tetracarbonsäure waschen, beispielsweise mit Wasser oder mit bei Zimmertemperatur flüssiger Carbonsäure wie beispielsweise Ameisensäure oder Essigsäure, insbesondere mit Eisessig, und trocknen.

Man kann die Reaktionsmischung partiell mit Base neutralisieren und dadurch Salze bilden, beispielsweise mit basischen Alkalimetallsalzen. Geeignete basische Alkalimetallsalze sind die Hydrogencarbonate, Carbonate und insbesondere die Hydroxide von Alkalimetallen wie Lithium, Rubidium und insbesondere Kalium und Natrium. Wünscht man die Reaktionsmischung partiell mit Base zu neutralisieren, so neutralisiert man üblicherweise gegebenenfalls verbliebene Salpetersäure und maximal eine Carboxylgruppe von erfindungsgemäß hergestellter Tetracarbonsäure der allgemeinen Formel I.

Durch das erfindungsgemäße Verfahren erhält man beispielsweise besonders reine Tetracarbonsäure der allgemeinen Formel I.

Ein weiterer Gegenstand der vorliegenden Erfindung sind Gemische von Tetracarbonsäure der allgemeinen Formel I bzw, ihres Salzes 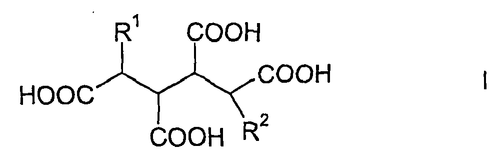 wobei die Variablen wie folgt definiert sind:
- R¹, R²: verschieden oder vorzugsweise gleich und gewählt aus Phenyl,
C₁-C₁₀-Alkyl, verzweigt oder bevorzugt unverzweigt, wie Methyl, Ethyl n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, 2-Ethylhexyl, n-Nonyl, n-Decyl, besonders bevorzugt C₁-C₄-Alkyl wie Methyl, Ethyl, n-Propyl, n-Butyl,
und ganz besonders bevorzugt Wasserstoff,
und im Bereich von 0,1 ppm bis 0,1 Gew.-% Verbindung der allgemeinen Formel IV 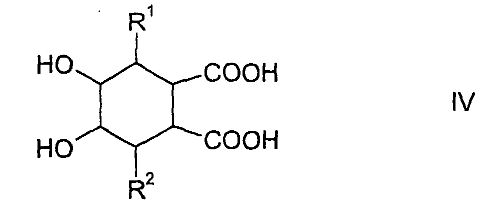 in der die Variablen wie oben stehend definiert sind.

Tetracarbonsäure der allgemeinen Formel I und vorstehend charakterisierte Gemische lassen sich besonders bequem durch das erfindungsgemäße Verfahren erhalten.

Erfindungsgemäß hergestellte Tetracarbonsäure der allgemeinen Formel 1 und vorstehend charakterisierte Gemische zeigen bei Erhitzen auf beispielsweise Temperaturen im Bereich von 120 bis 145°C auch für mehr als 30 Minuten keine mit bloßem Auge wahrnehmbare Verfärbung.

Erfindungsgemäß hergestellte Tetracarbonsäure der allgemeinen Formel I und vorstehend charakterisierte Gemische eignen sich zur Behandlung von Textilien , insbesondere Baumwolle. Ganz besonders gut eignen sich erfindungemäß hergestellte Tetracarbonsäure der allgemeinen Formel I und vorstehend charakterisierte Gemische zur Knitterfestausrüstung von Textilien wie beispielsweise Baumwolle. Ein weiterer Gegenstand der vorliegenden Erfindung ist daher die Verwendung von erfindungsgemäß hergestellten Tetracarbonsäuren der allgemeinen Formel I und vorstehend charakterisierten Gemischen zur Knitterfestausrüstung von Textil. Ein weiterer Gegenstand der vorliegenden Erfindung ist ein Verfahren zur Knitterfestausrüstung von Textilien unter Verwendung von erfindungsgemäßen hergestellten Tetracarbonsäuren der allgemeinen Formel I und vorstehend charakterisierten Gemischen.

Unter Textilien sind zwei- oder dreidimensionale Substrate aus faserförmigem Material zu verstehen, beispielsweise Fasern, Garne, Zwirne, Maschenware, Webware, Nonwovens und konfektionierte Ware aus cellulosehaltigem Material, beispielsweise Baumwolle, Jute, Flachs, Hanf und Ramie sowie Baumwollmischgewebe mit Polyester, modifiziertem Polyester, Polyamid, Polyacrylnitril, Triacetat, Acetat, Polycarbonat, Polypropylen, Polyvinylchlorid, Viskose, Seide.

So ist es beispielsweise möglich, eine oder mehrere erfindungsgemäß hergestellte Tetracarbonsäure der allgemeinen Formel I bzw. ein oder mehrere vorstehend charakterisierten Gemische in Mengen von 0,5 bis 10 mol-%, bezogen auf Anhydroglucose in Baumwolle, auf entsprechende Baumwolle oder Baumwolle/Kunstfaser-Mischgewebe einwirken zu lassen. Ein derartiges Einwirkenlassen kann in Gegenwart eines Vernetzungskatalysators wie beispielsweise NaH₂PO₂ geschehen. Anschließend kann man bei Temperaturen im Bereich von beispielsweise 120 bis 180°C, bevorzugt 145 bis 165°C trocknen, insbesondere bei Vermeidung von mechanischer Spannung. Erfindungsgemäß hergestellte Tetracarbonsäuren der allgemeinen Formel I und vorstehend charakterisierte Gemischen zur Knitterfestausrüstung von Textilien verursachen eine Vernetzung der Cellulosefasern und somit eine verbesserte Knitterfreiheit.

Es ist auch möglich, erfindungsgemäß hergestellte Tetracarbonsäuren der allgemeinen Formel I bzw. vorstehend charakterisierte Gemische in Kombination mit Oligomaleinsäurephosphonaten zur Knitterfestausrüstung von Textilien nach Verfahrensbedingungen einzusetzen, die beispielsweise aus WO 03/33811 im Grunde bekannt sind.

Die Erfindung wird durch Arbeitsbeispiele erläutert.

Vorbemerkung: *cis*-Δ⁴-Tetrahydrophthalsäureanhydrid wurde durch Umsetzung von Maleinsäureanhydrid mit Butadien nach GB 1,032,883 hergestellt.

### 1. Umsetzung von cis-Δ⁴-Tetrahydrophthalsäure mit Isolierung der Zwischenstufe nach Schritt (a)

In einem 500-ml-Dreihalskolben mit Rührer, Rückflusskühler und Innenthermometer wurden 91,4 g *cis*-Δ⁴-Tetrahydrophthalsäureanhydrid und 59,1 g Wasser vorgelegt. Unter Rühren wurde auf eine Innentemperatur von 95 bis 100°C erhitzt. Man rührte 30 Minuten bei 95°C und kühlte danach auf 80°C ab. Danach gab man 82,5 g 50 Gew.-% wässrige H₂O₂-Lösung innerhalb von 15 Minuten zu und rührte danach vier Stunden bei 80°C. Danach destillierte man am Rotationsverdampfer bis zur Trockene ab. Es verblieb 118 g *trans*-4,5-Dihydroxyhexahydrophthalsäure als farbloser Feststoff.

In einem 500-ml-Vierhalskolben mit Rührer, Tropftrichter, Rückflusskühler, Abgang zu einem mit Natronlauge gefüllten Abgaswäscher und Innenthermometer wurden 112 g 65 Gew.-% wässrige Salpetersäure und 22 mg Ammoniummetavanadat NH₄VO₃ vorgelegt und unter Rühren auf 65°C erwärmt. Die wie vorstehend erhaltene *trans*-4,5-Dihydroxyhexahydrophthalsäure wurde portionsweise zugegeben. Es entwickelten sich rotbraune nitrose Gase. Man rührte 4 Stunden bei 65°C nach. Die so erhaltene Reaktionsmischung wurde danach auf Zimmertemperatur abgekühlt. Meso-1,2,3,4-Butantetracarbonsäure schied sich als farbloser Feststoff ab, der abfiltriert, mit Eisessig gewaschen und im Vakuum getrocknet wurde. Die Ausbeute betrug 80%, bezogen auf *trans*-4,5-Dihydroxyhexahydrophthalsäure. Auch mit Hilfe von Gaschromatographie ließen sich keine Verunreinigungen detektieren.

### 2. Umsetzung von cis-Δ⁴-Tetrahydrophthalsäure ohne Isolierung der Zwischenstufe nach Schritt (a)

In einem 500-ml-Vierhalskolben mit Rührer, Tropftrichter, Rückflusskühler, Innenthermometer und Abgang zu einem mit Natronlauge gefüllten Abgaswäscher wurden 91,3 g *cis*-Δ⁴-Tetrahydrophthalsäureanhydrid und 59,6 g Wasser vorgelegt. Unter Rühren wurde auf eine Innentemperatur von 95 bis 100°C erhitzt. Man rührte 30 Minuten bei 95°C und kühlte danach auf 80°C ab. Danach gab man 81,8 g 50 Gew.-% wässrige H₂O₂-Lösung innerhalb von 15 Minuten zu und rührte danach vier Stunden bei 80°C. Danach kühlte man auf 65°C ab und gab 22 mg Ammoniummetavanadat zu. Unter Rühren gab man 345 g 65 Gew.-% wässrige Salpetersäure zu. Es entwickelten sich rotbraune nitrose Gase. Man rührte 4 Stunden bei 65°C nach. Die so erhaltene Reaktionsmischung wurde danach auf Zimmertemperatur abgekühlt. Meso-1,2,3,4-Butantetracarbonsäure schied sich als farbloser Feststoff ab, der abfiltriert, mit Eisessig gewaschen und im Vakuum getrocknet wurde. Die isolierte Ausbeute betrug 56%, bezogen auf *cis*-Δ⁴-Tetrahydrophthalsäureanhydrid. Auch mit Hilfe von Gaschromatographie ließen sich keine Verunreinigungen detektieren.

Durch Einengung der Mutterlaugen und eine weitere Kristallisation ließ sich zusätzliche Meso-1,2,3,4-Butantetracarbonsäure erhalten, die aber nicht mehr so rein war.

### 3. Umsetzung von cis-Δ⁴-Tetrahydrophthalsäure ohne Isolierung der Zwischenstufe nach Schritt (a), Abdestillieren von Wasser

In einem 500-ml-Vierhalskolben mit Rührer, Tropftrichter, Rückflusskühler, Innenthermometer und Abgang zu einem mit Natronlauge gefüllten Abgaswäscher wurden 91,3 g *cis*-Δ⁴-Tetrahydrophthalsäureanhydrid und 59,6 g Wasser vorgelegt. Unter Rühren wurde auf eine Innentemperatur von 95 bis 100°C erhitzt. Man rührte 30 Minuten bei 95°C und kühlte danach auf 80°C ab. Danach gab man 81,8 g 50 Gew.-% wässrige H₂O₂-Lösung innerhalb von 15 Minuten zu und rührte danach vier Stunden bei 80°C. Dabei destillierten etwa 5 g Wasser ab, der Kolbeninhalt blieb jedoch flüssig. Danach kühlte man auf 65°C ab und gab 56 mg Ammoniummetavanadat zu. Unter Rühren gab man 345 g 65 Gew.-% wässrige Salpetersäure zu. Es entwickelten sich rotbraune nitrose Gase. Man rührte 4 Stunden bei 65°C nach. Die so erhaltene Reaktionsmischung wurde danach auf Zimmertemperatur abgekühlt. Meso-1,2,3,4-Butantetracarbonsäure schied sich als farbloser Feststoff ab, der abfiltriert, mit Eisessig gewaschen und im Vakuum getrocknet wurde. Die Ausbeute betrug 81%, bezogen auf *cis*-Δ⁴-Tetrahydrophthalsäureanhydrid. Auch mit Hilfe von Gaschromatographie ließen sich keine Verunreinigungen detektieren.

### 4. Umsetzung von cis-Δ⁴-Tetrahydrophthalsäure mit Zugabe von Katalysator für Schritt (b) vor Schritt (a) ohne Isolierung der Zwischenstufe nach Schritt (a)

In einem 500-ml-Vierhalskolben mit Rührer, Tropftrichter, Destillationsaufsatz, Innenthermometer und Abgang zu einem mit Natronlauge gefüllten Abgaswäscher wurden 91,3 g *cis*-Δ⁴-Tetrahydrophthalsäureanhydrid, 26 mg Ammoniummetavanadat und 59,6 g Wasser vorgelegt. Unter Rühren wurde auf eine Innentemperatur von 95 bis 100°C erhitzt. Man rührte 30 Minuten bei 95°C und kühlte danach auf 80°C ab. Danach gab man 81,8 g 50 Gew.-% wässrige H₂O₂-Lösung innerhalb von 15 Minuten zu und rührte danach vier Stunden bei 80°C. Dabei destillierten etwa 5 g Wasser ab, der Kolbeninhalt blieb jedoch flüssig. Danach kühlte man auf 65°C ab. Unter Rühren gab man 345 g 65 Gew.-% wässrige Salpetersäure zu. Es entwickelten sich rotbraune nitrose Gase. Man rührte 4 Stunden bei 65°C nach. Die so erhaltene Reaktionsmischung wurde danach auf Zimmertemperatur abgekühlt. Meso-1,2,3,4-Butantetracarbonsäure schied sich als farbloser Feststoff ab, der abfiltriert, mit Eisessig gewaschen und im Vakuum getrocknet wurde. Die Ausbeute betrug 45%, bezogen auf *cis*-Δ⁴-Tetrahydrophthalsäureanhydrid. Auch mit Hilfe von Gaschromatographie ließen sich keine Verunreinigungen detektieren.

Durch Einengung der Mutterlauge und eine weitere Kristallisation ließ sich zusätzliche Meso-1,2,3,4-Butantetracarbonsäure erhalten, die aber nicht mehr so rein war.

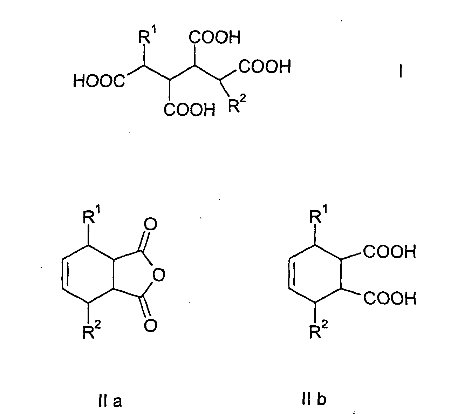

## Patentansprüche

1. Verfahren zur Herstellung von Tetracarbonsäuren der allgemeinen Formel I 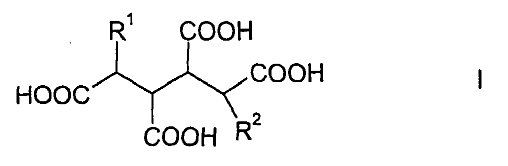 oder ihrer Salze, **dadurch gekennzeichnet, dass** man mindestens eine Verbindung der allgemeinen Formel II a oder II b 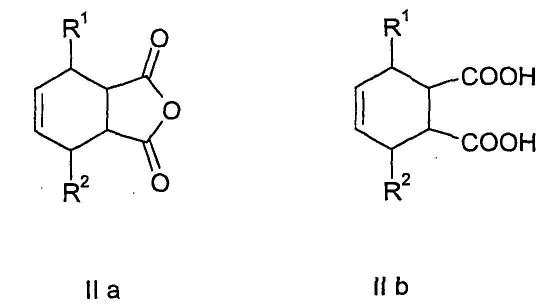
(a) zunächst mit mindestens einem Epoxidierungsreagenz umsetzt und
(b) danach mit Salpetersäure oder mindestens einem Stickoxid oxidiert,
wobei die Variablen wie folgt definiert sind:
R¹, R² gleich oder verschieden und gewählt aus Wasserstoff, Phenyl und C₁-C₁₀-Alkyl.

2. Verfahren nach Anspruch 1, **dadurch gekennzeichnet, dass** in Formel I, Formel II a bzw. II b R¹, R² jeweils gleich und Wasserstoff sind.

3. Verfahren nach Anspruch 1 oder 2, **dadurch gekennzeichnet, dass** man als Epoxidierungsreagenz in Schritt (a) Wasserstoffperoxid wählt.

4. Verfahren nach eine der Ansprüche 1 bis 3, **dadurch gekennzeichnet, dass** man Schritt (a) ohne Verwendung eines Katalysators durchführt.

5. Verfahren nach einem der Ansprüche 1 bis 4, **dadurch gekennzeichnet, dass** man Schritt (b) in Gegenwart eines Katalysators durchführt.

6. Verfahren nach einem der Ansprüche 1 bis 5, **dadurch gekennzeichnet, dass** man Schritt (b) in Gegenwart eines Katalysators durchführt, der Vanadium enthält.

7. Verfahren nach einem der Ansprüche 1 bis 6, **dadurch gekennzeichnet, dass** man Schritt (a) und (b) ohne Isolierung von Zwischenprodukt aneinander anschließt.

8. Verfahren nach einem der Ansprüche 1 bis 7, **dadurch gekennzeichnet, dass** man nach Durchführung von Schritt (a) und (b) Tetracarbonsäure der allgemeinen Formel I in ihr Salz überführt.

9. Verfahren nach Anspruch 8, **dadurch gekennzeichnet, dass** es sich bei dem Salz um ein Monoalkalimetallsalz handelt.

10. Verfahren nach einem der Ansprüche 1 bis 9, **dadurch gekennzeichnet, dass** man Schritt (b) bei Temperaturen im Bereich von 20 bis 70°C durchführt.

## Claims

1. A process for preparing tetracarboxylic acids of the general formula I 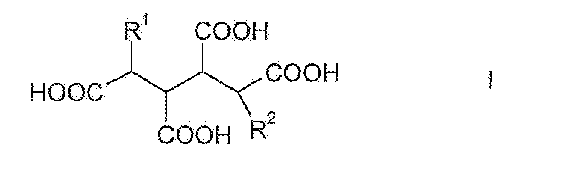 or salts thereof, wherein at least one compound of the general formula II a or II b 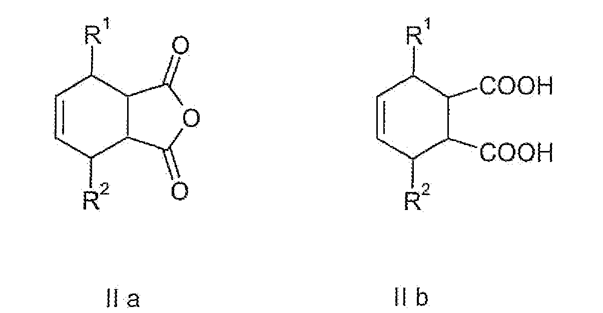
(a) is first reacted with at least one epoxidation reagent and
(b) then oxidized with nitric acid or at least one nitrogen oxide,
where the variables are defined as follows:
R¹, R² are the same or different and are selected from hydrogen, phenyl and C₁-C₁₀-alkyl.

2. The process according to claim 1, wherein R¹, R² in formula I, formula II a and II b are each the same and are hydrogen.

3. The process according to claim 1 or 2, wherein the epoxidation reagent selected in step (a) is hydrogen peroxide.

4. The process according to any of claims 1 to 3, wherein step (a) is carried out without the use of a catalyst.

5. The process according to any of claims 1 to 4, wherein step (b) is carried out in the presence of a catalyst.

6. The process according to any of claims 1 to 5, wherein step (b) is carried out in the presence of a catalyst which comprises vanadium.

7. The process according to any of claims 1 to 6, wherein step (b) follows (a) without isolation of intermediate.

8. The process according to any of claims 1 to 7, wherein, after step (a) and (b) have been carried out, tetracarboxylic acid of the general formula I is converted to its salt.

9. The process according to claim 8, wherein the salt is a mono(alkali metal) salt.

10. The process according to any of claims 1 to 9, wherein step (b) is carried out at temperatures in the range from 20 to 70°C.

## Revendications

1. Procédé pour la préparation d'acides tétracarboxyliques de formule générale I : 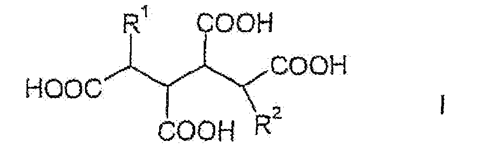 ou de leurs sels, **caractérisé en ce qu'**on transforme au moins un composé de formule générale II a ou II b 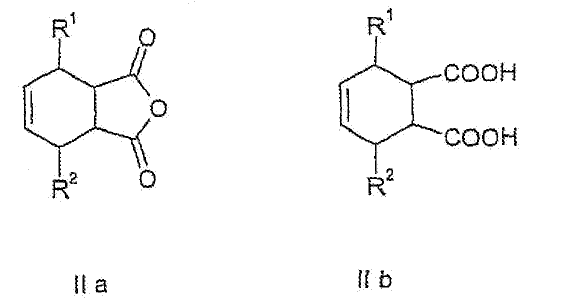
(a) d'abord avec au moins un réactif d'époxydation et
(b) ensuite avec de l'acide nitrique ou au moins un oxyde d'azote,
les variables étant définies comme suit :
R¹, R² sont identiques ou différents et choisis parmi hydrogène, phényle et C₁-C₁₀-alkyle.

2. Procédé selon la revendication 1, **caractérisé en ce que** dans la formule I, la formule II a ou II b, R¹, R² sont à chaque fois identiques et représentent hydrogène.

3. Procédé selon la revendication 1 ou 2, **caractérisé en ce qu'**on choisit comme réactif d'époxydation dans l'étape (a) du peroxyde d'hydrogène.

4. Procédé selon l'une quelconque des revendications 1 à 3, **caractérisé en ce qu'**on réalise l'étape (a) sans utiliser de catalyseur.

5. Procédé selon l'une quelconque des revendications 1 à 4, **caractérisé en ce qu'**on réalise l'étape (b) en présence d'un catalyseur.

6. Procédé selon l'une quelconque des revendications 1 à 5, **caractérisé en ce qu'**on réalise l'étape (b) en présence d'un catalyseur, qui contient du vanadium.

7. Procédé selon l'une quelconque des revendications 1 à 6, **caractérisé en ce** l'étape (a) et l'étape (b) se suivent sans isolement du produit intermédiaire.

8. Procédé selon l'une quelconque des revendications 1 à 7, **caractérisé en ce qu'**on transforme, après la réalisation de l'étape (a) et de l'étape (b) les acides tétracarboxyliques de formule générale I en leur sel.

9. Procédé selon la revendication 8, **caractérisé en ce qu'**il s'agit, pour le sel, d'un sel de métal alcalin monovalent.

10. Procédé selon l'une quelconque des revendications 1 à 9, **caractérisé en ce qu'**on réalise l'étape (b) à des températures dans la plage de 20 à 70°C.
